# EUROPEAN PATENT SPECIFICATION

(11) **EP 0 563 791 B1**
(45) Date of publication and mention of the grant of the patent: **22.01.1997**
(21) Application number: 93104930.8
(22) Date of filing: 25.03.1993
(51) Int. Cl.: C07C 69/63, C07C 67/08, C07C 67/14, G11B 5/71, G11B 5/72, C07C 69/65

(54) **Fluorine-containing alkyl-succinic acid diester, process for preparing the same and use thereof**
Fluor enthaltende Alkylbernsteinsäurediester, Verfahren zu ihrer Herstellung und ihre Anwendung
Diesters contenant du fluor de l'acide alkylsuccinique, leurs procédés de préparation et leur utilisation

(30) Priority: 31.03.1992 JP 76692/92
(43) Date of publication of application: 06.10.1993
(73) Proprietor: MATSUSHITA ELECTRIC INDUSTRIAL CO., LTD., Kadoma-shi, Osaka-fu, 571 (JP)
(72) Inventor: Kai, Yoshiaki, Osaka-u (JP); Mizuno, Naoko, Hirakata-shi, Osaka-fu (JP)
(74) Representative: Eisenführ, Speiser & Partner

(56) References cited:
- EP-A- 0 473 871
- DATABASE WPI, Week 9013, Derwent Publications Ltd., London, GB; AN 90-096291 & JP-A-2 049 218
- DATABASE WPI, Week 8435, Derwent Publications Ltd., London, GB; AN 84-216525 & JP-A-59 127 230
- DATABASE WPI, Week 8316, Derwent Publications Ltd., London, GB; AN 83-37733K & JP-A-58 041 438

## Description

The present invention relates to a fluorine-containing alkylsuccinic acid diester which is useful as a lubricant of a precision instrument or part with which highly precise lubrication is required, a surfactant, a mold release agent, or a rust preventive, a process for preparing said diester and a magnetic recording medium which has a lubricating layer comprising said diester.

With miniaturization and tendency for high accuracy of a machine or apparatus or a part, a lubricating type of a sliding part thereof has been shifted from fluid lubrication to boundary lubrication. In particular, with electronic equipments or parts such as a VTR, a magnetic disc and the like, a ferromagnetic metal thin film is used for the increase of a recording density. Then, highly accurate lubrication is required for sliding contact of a magnetic tape or disc against a magnetic head. For example, in the case of a metal deposition tape or hard disc, a lubricant layer is formed on a magnetic layer in a thickness of only several ten Å to reduce a spacing loss between the magnetic recording medium and the magnetic head as much as possible and achieve a large output, while maintaining durability and reliability of the tape or disc. To this end, it is highly desired to develop an organic compound having good lubricity as a material for forming such lubricant layer.

As a lubricant for a metal thin film type magnetic recording medium, a fluoroalkylether group-containing monocarboxylic acid of the formula:

R₄COOH (1)

wherein R₄ is a fluoroalkylether group is proposed since it has good compatibility with the metal thin film (see JP-A 41438/1983, 127230/1984 and 58414/1987).

Recently, a perfluoropolyether ester of a monocarboxylic acid of the formula:

R₅-(OOCR₆)ₙ (2)

wherein R₅ is a perfluoroalkylpolyether group, R₆ is an aliphatic alkyl group and n is a number of 1 to 6 is proposed as a lubricant (see JP-A 49218/1990).

However, the lubricant comprising the fluoroalkylether group-containing monocarboxylic acid (1) or the perfluoropolyether ester of monocarboxylic acid (2) will lose its lubricity in a high humidity atmosphere.

EP-A-O 473 871 discloses fluorine-containing compounds which are represented by the generel formula (R₁-Wₗ-)(R_{f}-Xₘ-R₂-Yₙ-R₃-)Z-R₄-COOH and wherein the various substituents and groups (R₁, R₂, R₃, R₄, R_{f}, W, X, Y and Z) are of such chemical nature that they lead to a monoester of a dicarboxylic acid or to a diester of a tricarboxylic acid. There is always one acid group remaining in the molekule. The monoesters are obtained by adding an aliphatic-succinic anhydride to an alcohol in an addition reaction taking place in an autoclave, while the diesters are produced by reacting an alkyl alcohol stepwise and separately with an anhydride chloride. The compounds of this document are reported to be useful as lubricants for precision machines and precision parts which require high-precision lubrication.

One object of the present invention is to provide a novel fluorine-containing alkylsuccinic acid diester which is useful as a lubricant suffering from no or less deterioration of lubricity in an atmosphere of from low humidity to high humidity.

Another object of the present invention is to provide a process for preparing the novel fluorine-containing alkylsuccinic acid diester of the present invention.

A further object of the present invention is to provide a magnetic recording medium having a lubricating layer which contains the novel fluorine-containing alkylsuccinic acid diester of the present invention as a lubricant.

These objects are attained by a diester, a magnetic recording medium, and a process for preparing the diester as defined in the claims.

According to a first aspect of the present invention, there is provided a fluorine-containing alkylsuccinic acid diester of the formula: 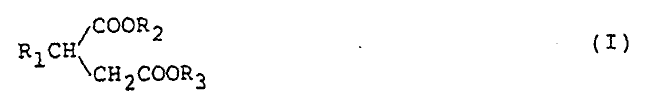 wherein R₁ is an aliphatic alkyl or alkenyl group, and one of R₂ and R₃ is a fluoroalkylether group and the other is a fluoroalkyl group, a fluoroalkenyl group, a fluorophenyl group, an aliphatic alkyl group or an aliphatic alkenyl group.

According to a second aspect of the present invention, there is provided a magnetic recording medium comprising a nonmagnetic substrate, a ferromagnetic metal layer formed thereon and a lubricating layer comprising a fluorine-containing alkylsuccinic acid diester of the formula (I). The magnetic recording medium may optionally have a protective layer between the ferromagnetic metal layer and the lubricating layer.

According to a third aspect of the present invention, there is provided a process (first process) for preparing a fluorine-containing alkylsuccinic acid diester of the formula (I) comprising steps of
addition reacting an aliphatic alkylsuccinic anhydride or aliphatic alkenylsuccinic anhydride with a fluoroalkylether group-containing alcohol in the presence of an acid catalyst to obtain a monoester and
esterifying said monoester with at least one compound selected from the group consisting of a fluoroalkyl group-containing alcohol, a fluoroalkenyl group-containing alcohol, a fluorophenyl group-containing alcohol, an aliphatic alkyl alcohol and an aliphatic alkenyl alcohol in the presence of an acid catalyst to obtain the diester.

According to a fourth aspect of the present invention, there is provided a process (second process) for preparing a fluorine-containing alkylsuccinic acid diester of the formula (I) comprising steps of
addition reacting an aliphatic alkylsuccinic anhydride or aliphatic alkenylsuccinic anhydride with at least one compound selected from the group consisting of a fluoroalkyl group-containing alcohol, a fluoroalkenyl group-containing alcohol, a fluorophenyl group-containing alcohol, an aliphatic alkyl alcohol and an aliphatic alkenyl alcohol to obtain a monoester and
esterifying said monoester with a fluoroalkylether group-containing alcohol in the presence of an acid catalyst to obtain the diester.

According to a fifth aspect of the present invention, there is provided a process (third process) for preparing a fluorine-containing alkylsuccinic acid diester of the formula (I) comprising steps of
addition reacting an aliphatic alkylsuccinic anhydride or aliphatic alkenylsuccinic anhydride with a fluoroalkylether group-containing alcohol in the presence of an acid catalyst to obtain a monoester,
chlorinating said monoester to obtain a chlorinated carboxylic acid and
reacting said chlorinated carboxylic acid with at least one compound selected from the group consisting of a fluoroalkyl group-containing alcohol, a fluoroalkenyl group-containing alcohol, a fluorophenyl group-containing alcohol, an aliphatic alkyl alcohol and an aliphatic alkenyl alcohol in the presence of a base catalyst.

According to a sixth aspect of the present invention, there is provided a process (fourth process) for preparing a fluorine-containing alkylsuccinic acid diester of the formula (I) comprising steps of
addition reacting an aliphatic alkylsuccinic anhydride or aliphatic alkenylsuccinic anhydride with at least one compound selected from the group consisting of a fluoroalkyl group-containing alcohol, a fluoroalkenyl group-containing alcohol, a fluorophenyl group-containing alcohol, an aliphatic alkyl alcohol and an aliphatic alkenyl alcohol to obtain a monoester,
chlorinating said monoester to obtain a chlorinated carboxylic acid and
reacting said chlorinated carboxylic acid with a fluoroalkylether group-containing alcohol in the presence of a base catalyst to obtain the diester.

The aliphatic alkyl or alkenyl group for substituent R₁ in the formula (I) has preferably 6 to 30 carbon atoms, more preferably 10 to 24 carbon atoms. When the number of carbon atoms in the aliphatic alkyl or alkenyl group is less than 6 or larger than 30, the diester may have decreased lubricity.

The fluoroalkylether group for the substituent R₂ or R₃ has preferably 5 to 50 carbon atoms, more preferably 8 to 30 carbon atoms. The fluoroalkyl ether group has at least one fluorine atom and it can be a perfluoroalkylether group. When the number of carbon atoms in the fluoroalkylether group is less than 5 or larger than 50, the diester may have deteriorated lubricity.

The fluoroalkyl or fluoroalkenyl group or the aliphatic alkyl or alkenyl group has preferably 30 or less carbon atoms, more preferably 20 or less carbon atoms. When the number of carbon atoms exceeds 30, the diester may have deteriorated lubricity. The fluorophenyl group may contain 1 to 5 fluorine atoms.

The fluorine-containing alkylsuccinic acid diester of the present invention is preferably prepared by either one of the processes of the present invention.

In the first and second processes, the addition reaction for the preparation of monoester and the esterification for the preparation of diester are carried out in the presence of a hydrophobic solvent in which the reactants and the monoester are dissolved and which can set a reaction temperature in a range between 40°C and 150°C, preferably 60°C and 120°C except alcohols or esters. Specific examples of such solvent are n-heptane, benzene, toluene, isooctane, cyclohexane, ethylene chloride, isopropyl ether, etc.

As the acid catalyst, any acid catalyst which can be used in a conventional esterification may be used. Examples of the acid catalyst are inorganic acids (e.g. sulfuric acid, hydrochloric acid, etc.), organic acids (e.g. aromatic sulfonic acids, etc.) and Lewis acids (e.g. boron fluoride etherate, etc.).

In the first process, the acid catalyst is used in an amount of 2 to 10 % by weight based on the total weight of the aliphatic alkyl- or alkenylsuccinic anhydride and the fluoroalkyl group-containing alcohol in the preparation of monoester, and 1 to 3 % by weight based on the total weight of the monoester and the alcohol in the preparation of diester.

In the second process, the acid catalyst is used in an amount of 2 to 10 % by weight based on the total weight of the monoester and the fluoroalkylether group-containing alcohol.

In the first process, a molar ratio of the aliphatic alkyl- or alkenylsuccinic anhydride to the fluoroalkylether group-containing alcohol is preferably from 2:1 to 1:2, more preferably from 3:2 to 2:3. The molar ratio of the monoester to at least one compound selected from the group consisting of a fluoroalkyl group-containing alcohol, a fluoroalkenyl group-containing alcohol, a fluorophenyl group-containing alcohol, an aliphatic alkyl alcohol and an aliphatic alkenyl alcohol is preferably from 2:1 to 1:2, more preferably from 3:2 to 2:3.

The above molar ratios can apply to molar ratios in the second process.

In the third and fourth processes, the same solvent as used in the above processes may be used. Also, the same catalyst as used in the above processes may be used in the same amount.

In the chlorination step, the monoester is chlorinated with a chlorinating agent such as phosphoryl chloride, thionyl chloride, phosphorus pentachloride, phosphorus trichloride, etc.

The chlorination agent is used in an amount of 3 to 30 equivalents per one mole of the monoester.

The chlorination of monoester can be carried out in a liquid phase in the presence or absence of a solvent at a temperature of 10°C to a refluxing temperature of the reaction mixture. When the solvent is used, n-hexane, cyclohexane, benzene, chloroform, ethyl ether, and the like may be used.

As the base catalyst, any base catalyst which can be used in a conventional esterification may be used. Examples of the base catalyst are pyridine, triethylamine, zinc chloride, iodide and the like.

The base catalyst is used in an amount of 10 to 100 % by weight of the chlorinated carboxylic acid.

The molar ratios between the reagents in the third and fourth processes are analogous to those in the first and second processes.

The magnetic recording medium can be produced by any of conventional methods using the diester of the present invention as a lubricant. For example, a ferromagnetic layer is formed on a non-magnetic substrate by a known method such as sputtering. Then, a lubricating layer comprising the diester of the present invention is coated on the ferromagnetic layer with or without a protective layer between the ferromagnetic layer and the lubricating layer.

In addition to the diester of the present invention, the lubricating layer may optionally contain one or more known additives such as other lubricant, a rust preventive, etc.

The diester of the present invention is coated on the ferromagnetic layer in an amount of 0.05 to 100 mg/m², preferably 0.1 to 50 mg/m².

As the other lubricant and the rust preventive, fluorine-containing ones are preferred. In particular, liquid fluorine-containing compounds are preferred.

An amount of the other lubricant and/or the rust preventive is from 0 to 80 % by weight, preferably 0 to 70 % by weight of the total weight of the lubricating layer.

When the amount of the diester of the present invention is less than 20 % by weight, the effects of the present invention may not be achieved.

As the optionally formed protective layer, a carbonaceous layer is preferably used. The carbonaceous layer may be a amorphous, graphite or diamond-like carbon film or their laminated or mixed state formed by sputtering, plasma CVD and the like. A thickness of protective layer is usually from 50 to 500 Å.

The fluorine-containing alkylsuccinic acid diester of the formula (I) of the present invention contains one fluoroalkylether group and two aliphatic hydrocarbon end groups, namely the aliphatic alkyl or alkenyl end groups in a molecule, or one fluoroalkylether group, one fluorocarbon end group, namely one fluoroalkyl or fluoroalkenyl end group, and one aliphatic hydrocarbon end group in a molecule. Then, the fluoroalkylether group and the fluorocarbon group are exposed on the surface of ferromagnetic metal layer or protective layer so that they contribute to the decrease of a surface energy of such layer and provide a non-adhesive surface. Thereby, the fluoroalkylether group and the fluorocarbon group have protective functions of the surface of ferromagnetic metal layer or the magnetic head surface in a low humidity atmosphere. The fluoroalkylether group is better in this function than the fluorocarbon group. But, the fluoroalkylether group has a moisture-absorbing property in the high humidity atmosphere since it has plural hydrophilic ether linkages in the molecule. Since the fluorocarbon group and the aliphatic hydrocarbon group are hydrophobic, these groups contained in the same molecule will mask the hydrophilicity of the fluoroalkylether group. To make effective use of this masking effect, the fluorocarbon group and the aliphatic hydrocarbon group are bonded to the molecular end so that these groups will surround the fluoroalkylether group from both sides.

Since the aliphatic hydrocarbon group has a flexible carbon-carbon bond and its molecules are orientated by an intermolecular force among the adjacent molecules, it has a good lubricating property.

Since the fluorine-containing alkylsuccinic acid diester of the formula (I) of the present invention has the above end groups in good balance, it has a good lubricating property in any atmosphere from low humidity to high humidity.

### Example 1

In this Example, a compound of the formula: 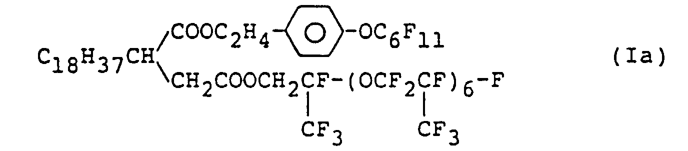 was prepared.

In a one liter flask equipped with a stirrer, octadecylsuccinic anhydride (35.3 g, 0.10 mole) of the formula: 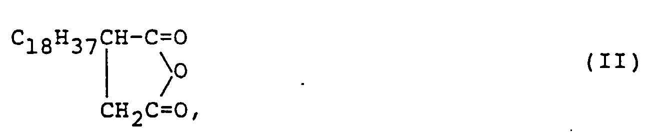 a fluoroalkylether group-containing alcohol (114.6 g, 0.10 mole) of the formula: 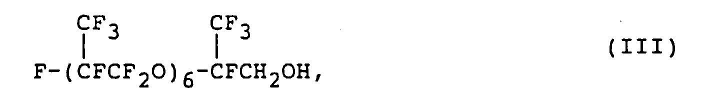 p-toluenesulfonic acid (hereinafter referred to as "PTS") (4.5 g corresponding to 3 % by weight of the whole raw materials) and n-heptane (500 ml) were charged and reacted under reflux for 24 hours.

After the reaction, the reaction mixture was washed with distilled water repeatedly till pH of the washing water decreased to 7 and dried over anhydrous sodium sulfate. After distilling n-heptane off, the residue was dissolved in benzene and cooled to -10°C to remove unreacted octadecylsuccinic anhydride. Then, the mixture was dissolved in methanol and cooled to -10°C to remove the unreacted fluoroalkylether group-containing alcohol to obtain a monoester (102 g) of the formula:

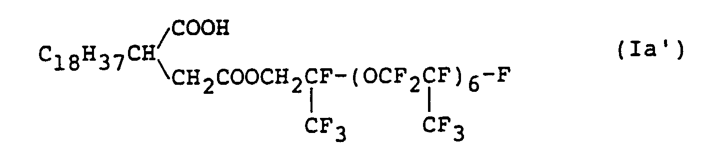

In a one liter flask equipped with a stirrer and a water condenser, the above monoester (Ia') (75.0 g, 0.05 mole), PTS (1.4 g corresponding to 1.5 % by weight of the monoester), benzene (300 ml) and a fluoroalkenyl group-containing alcohol (20.9 g, 0:05 mole) of the formula: 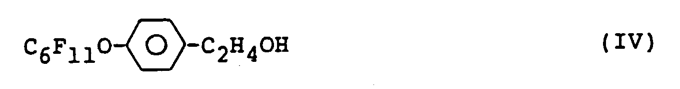 were charged and reacted under reflux for 24 hours.

After the reaction, the reaction mixture was washed with distilled water repeatedly till pH of the washing water decreased to 7 and dried over anhydrous sodium sulfate. After distilling benzene off, the residue was dissolved in methanol and cooled to -10°C to remove the unreacted monoester. Then, the mixture was dissolved in isopropanol and cooled to -10°C to remove the unreacted fluoroalkenyl group-containing alcohol to obtain a transparent colorless liquid (68 g), which was identified as the diester of the formula (Ia) containing neither raw materials nor by-products by IR, GPC and FD-MS.

IR: The peaks at 1775 cm⁻¹ (acid anhydride) and 3330 cm⁻¹ (alcohol) disappeared while the peak at 1760 cm⁻¹ (ester) appeared.

GPC: None of fluoroalkylether group-containing alcohol, fluoroalkenyl group-containing alcohol, octadecylsuccinic anhydride and the monoester was detected.

FD-MS: main peak at m/e of 1899.

When an aliphatic alkenylsuccinic anhydride is used in place of octadecylsuccinic anhydride in the above procedure, a corresponding diester is obtained.

When a fluoroalkyl group-containing alcohol, a fluorophenyl group-containing alcohol, an aliphatic alkyl alcohol or an aliphatic alkenyl alcohol is used in place of the fluoroalkenyl group-containing alcohol in the above procedure, a corresponding diester is obtained.

### Example 2

In this Example, the diester of the formula (Ia) in Example 1 was prepared by another process.

In a one liter flask equipped with a stirrer, the octadecylsuccinic anhydride (II) (35.3 g, 0.10 mole), the fluoroalkenyl group-containing alcohol (IV) (41.8 g, 0.10 mole) and benzene (300 ml) were charged and reacted under reflux for 24 hours.

After the reaction, benzene was distilled off, and the residue was dissolved in isopropyl ether and cooled to -10°C to remove unreacted octadecylsuccinic anhydride. Then, the mixture was dissolved in methanol and cooled to -10°C to remove the unreacted fluoroalkenyl group-containing alcohol to obtain a monoester of the formula:

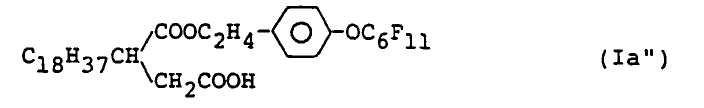

In a one liter flask equipped with a stirrer and a water condenser, the above monoester (Ia'') (38.5 g, 0.05 mole), PTS (2.9 g corresponding to 3.0 % by weight of the monoester), n-heptane (300 ml) and the fluoroalkylether group-containing alcohol (III) (57.3 g, 0.05 mole) were charged and reacted under reflux for 24 hours.

After the reaction, the reaction mixture was washed with distilled water repeatedly till pH of the washing water decreased to 7 and dried over anhydrous sodium sulfate. After distilling n-heptane off, the residue was dissolved in methanol and cooled to -10°C to remove the unreacted monoester. Then, the mixture was dissolved in isopropanol and cooled to -10°C to remove the unreacted fluoroalkylether group-containing alcohol to obtain a transparent colorless liquid (65 g), which was identified as the same diester (Ia) as obtained in Example 1.

### Example 3

In this Example, the diester of the formula (Ia) in Example 1 was prepared by a further process.

In a one liter flask equipped with a stirrer, the octadecylsuccinic anhydride (II) (35.3 g, 0.10 mole), the fluoroalkylether group-containing alcohol (III) (114.6 g, 0.10 mole), PTS (4.5 g corresponding 3 % by weight of of the whole raw materials) and n-heptane (500 ml) were charged and reacted under reflux for 24 hours.

After the reaction, the reaction mixture was treated in the same manner as in Example 1 to obtain the monoester (Ia') (102 g).

In a one liter flask equipped with a stirrer, the above monoester (Ia') (75.0 g, 0.05 mole) and thionyl chloride (60 g) were charged and reacted under reflux for 3 hours. After chlorination reaction, excessive thionyl chloride was distilled off under reduced pressure. To the residue, a solution of the fluoroalkenyl group-containing alcohol (IV) (20.9 g, 0.05 mole) in a mixture of pyridine (20 ml) and isopropyl ether (300 ml) was dropwise added over 2 hours while cooling with ice. After the addition of solution, the mixture was further stirred at room temperature for 4 hours to complete the reaction. Thereafter, the reaction mixture was washed with 5 % hydrochloric acid (300 ml) to remove excessive pyridine from the mixture.

The reaction mixture was washed with distilled water repeatedly till pH of the washing water decreased to 7 and dried over anhydrous sodium sulfate. After distilling isopropyl ether off, the residue was dissolved in methanol and cooled to -10°C to remove the unreacted monoester. Then, the mixture was dissolved in isopropanol and cooled to -10°C to remove the unreacted fluoroalkylether group-containing alcohol to obtain a transparent colorless liquid (72 g), which was identified as the same diester (Ia) as obtained in Example 1.

This process of Example 3 is characterized in that the monoester is chlorinated to obtain a chlorinated carboxylic acid and the chlorinated carboxylic acid is esterified in the presence of a base catalyst to obtain a diester. By this process, the yield reaches 90 % or higher, while the yields in Examples 1 and 2 are about 70 %.

In the above procedures, the fluoroalkylether group-containing alcohol was used in the monoesterifying step of the aliphatic alkylsuccinic anhydride and the fluoroalkenyl group-containing alcohol was used in the diesterifying step. These two alcohols can be used in the reverse order.

### Example 4

This Example illustrates the production of a magnetic recording medium according to the present invention.

As a non-magnetic substrate, there was used a polyester film having gently sloped protrusions, which were formed by silica minute particles and had an average height of 70 Å and an average diameter of 1 µm, in a density of several protrusions per 100 µm² and sharp protrusions formed from colloidal silica particles of 150 Å in diameter as nuclei and a UV curable epoxy resin as a binder in a density of 1 x 10⁷ protrusions per 1 mm², but relatively large protrusions formed by a residue of polymerization catalyst as few as possible.

On a surface of non-magnetic substrate, a Co-Ni ferromagnetic metal film having a thickness of 1000 Å and a Ni content of 20 % was formed in the presence of a very small amount of oxygen by a continuous inclined vapor deposition method. An oxygen content in the ferromagnetic metal layer was 5 atomic %.

On the surface of ferromagnetic metal film, the fluorine-containing alkylsuccinic acid diester of the formula (Ia) prepared in Example 1 was coated in an amount of 10 mg/m² to form a lubricating layer.

The film was then cut to a predetermined width to obtain a magnetic tape.

The magnetic tape was repeatedly run in a commercial video deck at 23°C, 10 %RH or at 40°C, 80 %RH, and output characteristics were measured. The number of runs was counted till the RF output decreased by 3 dB from the original RF output or till the output started to fluctuate.

### Example 5

In the same manner as in Example 4 except that the compound of the formula: 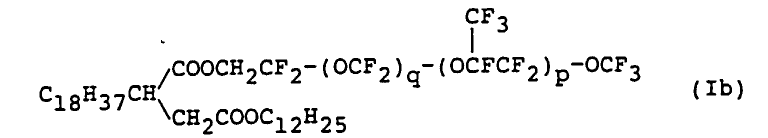 having a molecular weight of 2140 was used in place of the diester of the formula (Ia), a magnetic tape was produced and its property was measured.

### Example 6

In the same manner as in Example 4 except that a mixture of the diester of the formula (Ia) and a commercially available lubricant of the formula:

C₇F₁₅C₂H₄NHC₁₄H₂₉

in a weight ratio of 2:1 was used in place of the diester of the formula (Ia), a magnetic tape was produced and its property was measured.

### Example 7

In the same manner as in Example 4 except that a mixture of the diester of the formula (Ib) and a commercially available lubricant of the formula:

C₁₇H₃₅COOC₂H₄C₈F₁₇

in a weight ratio of 1:1 was used in place of the diester of the formula (Ia), a magnetic tape was prepared and its property was measured.

### Comparative Examples 1, 2 and 3

In the same manner as in Example 4 except that a compound of the formula: 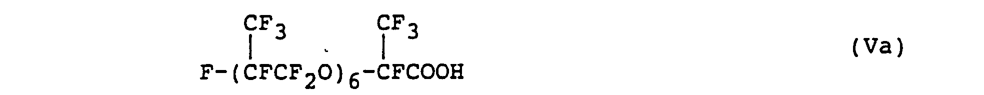 (Comparative Example 1), a compound of the formula: 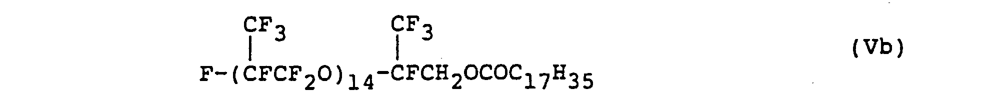 (Comparative Example 2) or a compound of the formula:

C₁₇H₃₅COOCH₂CF₂O(C₂F₄O)ₚ-(CF₂O)_{q}-CF₂CH₂OCOC₁₇H₃₅ (Vc)

(Comparative Example 3)
was used in place of the diester of the formula (Ia), a magnetic tape was produced and its property was measured.

The results are shown in Table 1.

**Table 1**

| Example No. | Number of Runs (times) | |
|---|---|---|
| | at 23°C, 10 %RH | at 40°C, 80 %RH |
| Comp. 1 | 120 | 80 |
| Comp. 2 | 130 | 110 |
| Comp. 3 | 90 | 120 |
| 4 | >200 | >200 |
| 5 | >200 | >200 |
| 6 | >200 | >200 |
| 7 | >200 | >200 |

From the results in Table 1, it is clear that the magnetic tapes having the lubricating layer comprising the fluorine-containing alkylsuccinic acid diester of the present invention had larger repeated running number in the low and high humidity than the magnetic tapes having the lubricating layer comprising the conventional lubricant.

### Example 8

As a non-magnetic substrate, there was used an aluminum alloy disc having a diameter of 95 mm and a thickness of 1.2 mm which was plated with a non-magnetic Ni-P alloy of 25 µm in thickness and texture finished to form protrusions having an average surface roughness of 50 Å and the maximum height of 300 Å.

On the non-magnetic Ni-P alloy layer, a Cr primer layer having a thickness of 1300 Å and a ferromagnetic metal layer of Co-Ni having a thickness of 600 Å were formed by sputtering.

On the ferromagnetic layer, a graphite protective layer having a thickness of 200 Å was formed by sputtering (Sample A), or a diamond-like carbon protective layer having a thickness of 50 Å was formed by the plasma CVD method (Sample B).

On the protective layer of each sample, a lubricating layer was formed by coating the fluorine-containing alkylsuccinic acid diester of the formula (Ia) in an amount of 10 mg/m² to produce a magnetic disc.

The magnetic disc was subjected to the CSS (contact start and stop) test at 23°C, 10 %RH or 40°C, 80 %RH. The number of the CSS tests was counted till a coefficient of friction on the surface of magnetic disc exceeded 1.0 or till the head crush occurred to evaluate the durability of the magnetic disc.

### Examples 9, 10 and 11

In the same manner as in Example 8 except that the diester of the formula (Ib) (Example 9), the lubricant mixture of Example 6 (Example 10) or the lubricant mixture of Example 7 (Example 11) was used in place of the diester of the formula (Ia), the magnetic disc was produced and subjected to the CSS test.

### Comparative Examples 4, 5 and 6

In the same manner as in Example 8 except that, in Comparative Examples 4, 5 and 6, the same commercially available lubricants as used in Comparative Examples 1, 2 and 3 were used, respectively in place of the diester of the formula (Ia), magnetic discs were produced and subjected to the CSS test.

The results are shown in Table 2.

**Table 2**

| Example No. | Sample No. | CSS number (times) | |
|---|---|---|---|
| | | 23°C, 10 %RH | 40°C, 80 %RH |
| Comp. 4 | A | 18,000 | 5,000 |
| Comp. 5 | A | 20,000*) | 8,000 |
| Comp. 6 | A | 12,000*) | 10,000 |
| 8 | A | >50,000 | >50,000 |
| 9 | B | >50,000 | >50,000 |
| 10 | A | >50,000 | >50,000 |
| 11 | A | >50,000 | >50,000 |

| | | | |
|---|---|---|---|
| Note: *) The head crush occurred. | | | |

From the results of Table 2, it is understood that the magnetic discs having the lubricating layer comprising the fluorine-containing alkylsuccinic acid diester of the present invention had better durability in the CSS test in the low and high humidity atmosphere than the magnetic tapes having the lubricating layer comprising the conventional lubricant.

With the following fluorine-containing alkylsuccinic acid diesters, the same results as in above Examples can be attained: 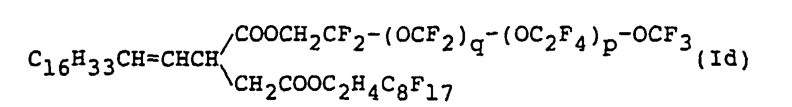 (Molecular weight of 1500) 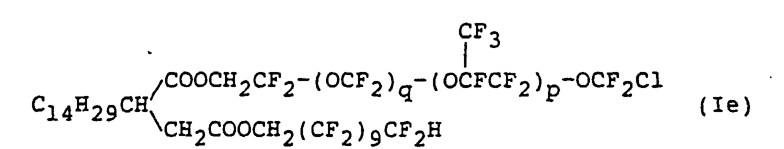 (Molecular weight of 1850) 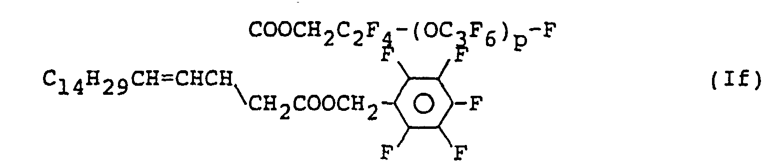 (Molecular weight of 3200) 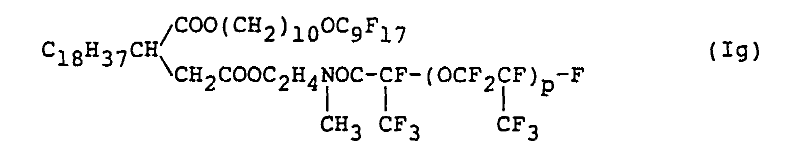 (Molecular weight of 2230) 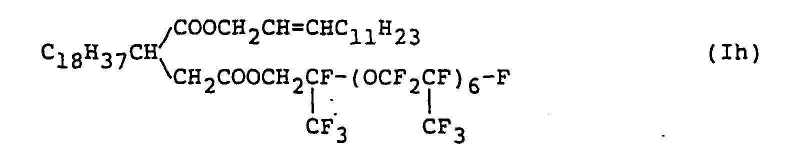 (Molecular weight of 1693)

## Claims

1. A fluorine-containing alkylsuccinic acid diester of the formula: 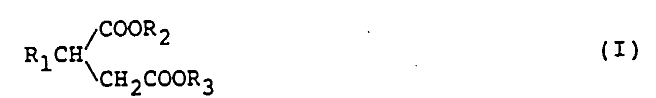 wherein R₁ is an aliphatic alkyl or alkenyl group, and one of R₂ and R₃ is a fluoroalkylether group and the other is a fluoroalkyl group, a fluoroalkenyl group, a fluorophenyl group, an aliphatic alkyl group or an aliphatic alkenyl group.

2. A magnetic recording medium comprising a non-magnetic substrate, a ferromagnetic metal layer formed thereon and a lubricating layer comprising a fluorine-containing alkylsuccinic acid diester of the formula (I) as claimed in claim 1.

3. The magnetic recording medium according to claim 2, which further comprises a protective layer between said ferromagnetic metal layer and said lubricating layer.

4. The magnetic recording medium according to claim 2, wherein said lubricating layer further comprises other lubricant.

5. A process for preparing a fluorine-containing alkylsuccinic acid diester of the formula (I) as claimed in claim 1 comprising steps of
addition reacting an aliphatic alkylsuccinic anhydride or aliphatic alkenylsuccinic anhydride with a fluoroalkylether group-containing alcohol in the presence of an acid catalyst to obtain a monoester and
esterifying said monoester with at least one compound selected from the group consisting of a fluoroalkyl group-containing alcohol, a fluoroalkenyl group-containing alcohol, a fluorophenyl group-containing alcohol, an aliphatic alkyl alcohol and an aliphatic alkenyl alcohol in the presence of an acid catalyst to obtain the diester.

6. A process for preparing a fluorine-containing alkylsuccinic acid diester of the formula (I) as claimed in claim 1 comprising steps of
addition reacting an aliphatic alkylsuccinic anhydride or aliphatic alkenylsuccinic anhydride with at least one compound selected from the group consisting of a fluoroalkyl group-containing alcohol, a fluoroalkenyl group-containing alcohol, a fluorophenyl group-containing alcohol, an aliphatic alkyl alcohol and an aliphatic alkenyl alcohol to obtain a monoester and
esterifying said monoester with a fluoroalkylether group-containing alcohol in the presence of an acid catalyst to obtain the diester.

7. A process for preparing a fluorine-containing alkylsuccinic acid diester of the formula (I) as claimed in claim 1 comprising steps of
addition reacting an aliphatic alkylsuccinic anhydride or aliphatic alkenylsuccinic anhydride with a fluoroalkylether group-containing alcohol in the presence of an acid catalyst to obtain a monoester,
chlorinating said monoester to obtain a chlorinated carboxylic acid and
reacting said chlorinated carboxylic acid with at least one compound selected from the group consisting of a fluoroalkyl group-containing alcohol, a fluoroalkenyl group-containing alcohol, a fluorophenyl group-containing alcohol, an aliphatic alkyl alcohol and an aliphatic alkenyl alcohol in the presence of a base catalyst.

8. A process for preparing a fluorine-containing alkylsuccinic acid diester of the formula (I) as claimed in claim 1 comprising steps of
addition reacting an aliphatic alkylsuccinic anhydride or aliphatic alkenylsuccinic anhydride with at least one compound selected from the group consisting of a fluoroalkyl group-containing alcohol, a fluoroalkenyl group-containing alcohol, a fluorophenyl group-containing alcohol, an aliphatic alkyl alcohol and an aliphatic alkenyl alcohol to obtain a monoester,
chlorinating said monoester to obtain a chlorinated carboxylic acid and
reacting said chlorinated carboxylic acid with a fluoroalkylether group-containing alcohol in the presence of a base catalyst to obtain the diester.

## Patentansprüche

1. Fluor enthaltender Alkylbernsteinsäurediester der Formel 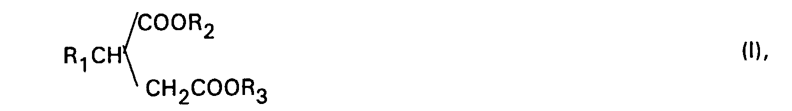 wobei R₁ eine aliphatische Alkyl- oder Alkenylgruppe bedeutet und eine der Gruppen R₂ und R₃ eine Fluoralkylethergruppe und die andere eine Fluoralkylgruppe, eine Fluoralkenylgruppe, eine Fluorphenylgruppe, eine aliphatische Alkylgruppe oder eine aliphatische Alkenylgruppe bedeutet.

2. Magnetisches Aufzeichnungsmedium, aufweisend ein nichtmagnetisches Substrat, eine darauf gebildete ferromagnetische Metallschicht und eine Schmierschicht, enthaltend einen Fluor enthaltenden Alkylbernsteinsäurediester der Formel (I) gemäß Anspruch 1.

3. Magnetisches Aufzeichnungsmedium nach Anspruch 2, weiter aufweisend eine Schutzschicht zwischen der ferromagnetischen Metallschicht und der Schmierschicht.

4. Magnetisches Aufzeichnungsmedium nach Anspruch 2, wobei die Schmierschicht außerdem andere Schmiermittel enthält.

5. Verfahren zum Herstellen eines Fluor enthaltenden Alkylbernsteinsäurediesters der Formel (I) gemäß Anspruch 1 mit den Schritten, daß man ein aliphatisches Bernsteinsäureanhydrid oder ein aliphatisches Alkenylbernsteinsäureanhydrid mit einem eine Fluoralkylethergruppe enthaltenden Alkohol in Gegenwart eines Säurekatalysators unter Addition zur Bildung eines Monoesters umsetzt und
den Monoester mit mindestens einer Verbindung in Gegenwart eines Säurekatalysators zur Bildung des Diesters verestert, wobei die Verbindung ausgewählt wird aus der Gruppe bestehend aus eine Fluoralkylgruppe enthaltenden Alkoholen, eine Fluoralkenylgruppe enthaltenden Alkoholen, eine Fluorphenylgruppe enthaltenden Alkoholen, aliphatischen Alkylalkoholen und aliphatischen Alkenylalkoholen.

6. Verfahren zum Herstellen eines Fluor enthaltenden Alkylbernsteinsäurediesters der Formel (I) gemäß Anspruch 1 mit den Schritten, daß man ein aliphatisches Alkylbernsteinsäureanhydrid oder ein aliphatisches Alkenylbernsteinsäureanhydrid mit mindestens einer Verbindung unter Addition zur Bildung eines Monoesters umsetzt, wobei die Verbindung ausgewählt wird aus der Gruppe bestehend aus eine Fluoralkylgruppe enthaltenden Alkoholen, eine Fluoralkenylgruppe enthaltenden Alkoholen, eine Fluorphenylgruppe enthaltenden Alkoholen, aliphatischen Alkylalkoholen und aliphatischen Alkenylalkoholen und
den Monoester mit einem eine Fluoralkylethergruppe enthaltenden Alkohol in Gegenwart eines Säurekatalysators zur Bildung des Diesters verestert.

7. Verfahren zum Herstellen eines Fluor enthaltenden Alkylbernsteinsäurediesters der Formel (I) gemäß Anspruch 1 mit den Schritten, daß man ein aliphatisches Bernsteinsäureanhydrid oder ein aliphatisches Alkenylbernsteinsäureanhydrid mit einem eine Fluoralkylethergruppe enthaltenden Alkohol in Gegenwart eines Säurekatalysators unter Addition zur Bildung eines Monoesters umsetzt,
den Monoester zur Bildung einer chlorierten Carbonsäure chloriert und die chlorierte Carbonsäure mit mindestens einer Verbindung in Gegenwart eines Basenkatalysators umsetzt, wobei die Verbindung ausgewählt wird aus der Gruppe bestehend aus eine Fluoralkylgruppe enthaltenden Alkoholen, eine Fluoralkenylgruppe enthaltenden Alkoholen, eine Fluorphenylgruppe enthaltenden Alkoholen, aliphatischen Alkylalkoholen und aliphatischen Alkenylalkoholen.

8. Verfahren zum Herstellen eines Fluor enthaltenden Alkylbernsteinsäurediesters der Formel (I) gemäß Anspruch 1 mit den Schritten, daß man ein aliphatisches Alkylbernsteinsäureanhydrid oder ein aliphatisches Alkenylbernsteinsäureanhydrid mit mindestens einer Verbindung unter Addition zur Bildung eines Monoesters umsetzt, wobei die Verbindung ausgewählt wird aus der Gruppe bestehend aus eine Fluoralkylgruppe enthaltenden Alkoholen, eine Fluoralkenylgruppe enthaltenden Alkoholen, eine Fluorphenylgruppe enthaltenden Alkoholen, aliphatischen Alkylalkoholen und aliphatischen Alkenylalkoholen,
den Monoester zur Bildung einer chlorierten Carbonsäure chloriert und die chlorierte Carbonsäure mit einem eine Fluoralkylethergruppe enthaltenden Alkohol in Gegenwart eines Basenkatalysators zur Bildung des Diesters umsetzt.

## Revendications

1. Diester d'acide alkylsuccinique contenant du fluor de formule : 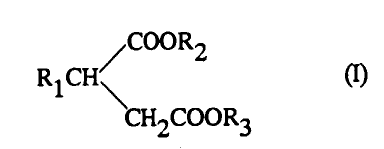 où R₁ est un groupe alkyle ou alcényle aliphatique, et l'un des groupes R₂ et R₃ est un groupe fluoroalkyléther et l'autre est un groupe fluoroalkyle, un groupe fluoroalcényle, un groupe fluorophényle, un groupe alkyle aliphatique ou un groupe alcényle aliphatique.

2. Milieu d'enregistrement magnétique comprenant un substrat non magnétique, une couche de métal ferromagnétique formée dessus et une couche lubrifiante comprenant un diester d'acide alkylsuccinique contenant du fluor selon la formule (I) et selon la revendication 1.

3. Milieu d'enregistrement magnétique selon la revendication 2, qui comprend en outre une couche protectrice entre ladite couche de métal ferromagnétique et ladite couche lubrifiante.

4. Milieu d'enregistrement magnétique selon la revendication 2, où ladite couche lubrifiante comprend en outre un autre lubrifiant.

5. Procédé de préparation d'un diester d'acide alkylsuccinique contenant du fluor de formule (I) selon la revendication 1 comprenant les étapes de
ajouter pour le faire réagir un anhydride d'acide alkylsuccinique aliphatique ou un anhydride d'acide alcénylsuccinique aliphatique à un alcool contenant un groupe fluoroalkyléther en présence d'un catalyseur acide pour donner un monoester, et
estérifier ledit monoester avec au moins un composé choisi dans le groupe constitué d'un alcool contenant un groupe fluoroalkyle, un alcool contenant un groupe fluoroalcényle, un alcool contenant un groupe fluorophényle, un alcool alkylaliphatique et un alcool alcénylaliphatique en présence d'un catalyseur acide pour obtenir le diester.

6. Procédé de préparation d'un diester d'acide alkylsuccinique contenant du fluor de formule (I) selon la revendication 1 comprenant les étapes de
ajouter pour le faire réagir un anhydride d'acide alkylsuccinique aliphatique ou un anhydride d'acide alcénylsuccinique aliphatique à au moins un composé choisi dans le groupe constitué d'un alcool contenant un groupe fluoroalkyle, un alcool contenant un groupe fluoroalcényle, un alcool contenant un groupe fluorophényle, un alcool alkylaliphatique et un alcool alcénylaliphatique pour donner un monoester, et
estérifier ledit monoester avec un alcool contenant un groupe fluoroalkyléther en présence d'un catalyseur acide pour donner le diester.

7. Procédé de préparation d'un diester d'acide alkylsuccinique contenant du fluor de formule (I) selon la revendication 1 comprenant les étapes de
ajouter pour le faire réagir un anhydride d'acide alkylsuccinique aliphatique ou un anhydride d'acide alcénylsuccinique aliphatique à un alcool contenant un groupe fluoroalkyléther en présence d'un catalyseur acide pour obtenir un monoester,
chlorer ledit monoester pour donner un acide carboxylique chloré et
faire réagir ledit acide carboxylique chloré avec au moins un composé choisi dans le groupe constitué d'un alcool contenant un groupe fluoroalkyle, un alcool contenant un groupe fluoroalcényle, un alcool contenant un groupe fluorophényle, un alcool alkylaliphatique et un alcool alcénylaliphatique en présence d'un catalyseur basique.

8. Procédé de préparation d'un diester d'acide alkylsuccinique contenant du fluor de formule (I) selon la revendication 1 comprenant les étapes de
ajouter pour le faire réagir un anhydride d'acide alkylsuccinique aliphatique ou un anhydride d'acide alcénylsuccinique aliphatique avec au moins un composé choisi dans le groupe constitué d'un alcool contenant un groupe fluoroalkyle, un alcool contenant un groupe fluoroalcényle, un alcool contenant un groupe fluorophényle, un alcool alkylaliphatique et un alcool alcénylaliphatique pour donner un monoester,
chlorer ledit monoester pour donner un acide carboxylique chloré et
faire réagir ledit acide carboxylique chloré avec un alcool contenant un groupe fluoroalkyléther en présence d'un catalyseur basique pour donner le diester.
